(19) **Europäisches Patentamt / European Patent Office / Office européen des brevets**

(11) **EP 3 327 435 B1**

(12) **FASCICULE DE BREVET EUROPEEN**

(45) Date de publication et mention de la délivrance du brevet:
**19.01.2022 Bulletin 2022/03**

(21) Numéro de dépôt: **17306535.0**

(22) Date de dépôt: **06.11.2017**

(51) Classification Internationale des Brevets (IPC):
**G01N 33/24** *(2006.01)* **E21B 49/08** *(2006.01)*
**G01N 33/00** *(2006.01)* **G01N 33/18** *(2006.01)*

(52) Classification Coopérative des Brevets (CPC):
**G01N 33/0036; G01N 33/004; G01N 33/005; G01N 33/18; G01N 33/24;** E21B 49/08

(54) **PROCEDE D'EXPLOITATION ET/OU DE SURVEILLANCE D'UN AQUIFERE COMPORTANT AU MOINS UN GAZ DISSOUS**

NUTZUNGS- UND/ODER ÜBERWACHUNGSVERFAHREN EINES MINDESTENS EIN GELÖSTES GAS ENHALTENDEN GRUNDWASSERLEITERS

PRODUCTION AND/OR MONITORING PROCESS OF AN AQUIFER COMPRISING AT LEAST ONE DISSOLVED GAS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2016 FR 1661559**

(43) Date de publication de la demande:
**30.05.2018 Bulletin 2018/22**

(73) Titulaire: **IFP Energies nouvelles
92852 Rueil-Malmaison Cedex (FR)**

(72) Inventeurs:
- **ROUCHON, Virgile
  92420 VAUCRESSON (FR)**
- **BEAUMONT, Valerie
  93100 MONTREUIL SOUS BOIS (FR)**
- **GARCIA, Bruno
  92200 NEUILLY SUR SEINE (FR)**
- **DURAND, Isabelle
  92500 RUEIL MALMAISON (FR)**

(74) Mandataire: **IFP Energies nouvelles
Département Propriété Industrielle
Rond Point de l'échangeur de Solaize
BP3
69360 Solaize (FR)**

(56) Documents cités:
- **GILFILLAN S M V ET AL: "The noble gas geochemistry of natural CO2 gas reservoirs from the Colorado Plateau and Rocky Mountain provinces, USA", GEOCHIMICA ET COSMOCHIMICA ACTA, PERGAMON PRESS, NEW YORK, NY, US, vol. 72, no. 4, 15 février 2008 (2008-02-15), pages 1174-1198, XP023782886, ISSN: 0016-7037, DOI: 10.1016/J.GCA.2007.10.009 [extrait le 2007-10-23]**
- **ARSLAN SEBNEM ET AL: "Analysis of groundwater dynamics in the complex aquifer system of Kazan Trona, Turkey, using environmental tracers and noble gases", HYDROGEOLOGY JOURNAL, SPRINGER BERLIN HEIDELBERG, BERLIN/HEIDELBERG, vol. 23, no. 1, 17 septembre 2014 (2014-09-17), pages 175-194, XP035428616, ISSN: 1431-2174, DOI: 10.1007/S10040-014-1188-Z [extrait le 2014-09-17]**
- **A. PAONITA ET AL: "Dissolved inert gases (He, Ne and N2) as markers of groundwater flow and degassing areas at Mt Etna volcano (Italy)", CHEMICAL GEOLOGY., vol. 443, 15 septembre 2016 (2016-09-15), pages 10-21, XP055385816, NL ISSN: 0009-2541, DOI: 10.1016/j.chemgeo.2016.09.018**

• **BRUNO GARCIA ET AL: "The CO2-vadose project: Numerical modeling to perform a geochemical monitoring methodology and baseline performance assessment for various geochemical variables (gas flux, gas composition, stable isotopes and noble gases) in the carbonate vadose zone", INTERNATIONAL JOURNAL OF GREENHOUSE GAS CONTROL, vol. 14, 1 mai 2013 (2013-05-01), pages 247-258, XP055386285, AMSTERDAM, NL ISSN: 1750-5836, DOI: 10.1016/j.ijggc.2013.01.029**

EP 3 327 435 B1

## Description

La présente invention concerne le domaine de l'exploration et de l'exploitation d'une formation souterraine comprenant un réservoir d'eau dans lequel au moins un gaz d'intérêt est dissous, ou encore la surveillance d'une telle formation souterraine.

De façon générale, la présente invention concerne la quantification d'un gaz d'intérêt présent sous une forme dissoute dans un réservoir d'eau, tel qu'un aquifère, une résurgence thermale, etc.

De façon générale, le gaz d'intérêt peut être un gaz injecté dans la formation en vue de son stockage souterrain (il pourra s'agir par exemple de $CO_2$, ou encore de méthane), un gaz issu d'une contamination industrielle (un gaz issus d'un stockage de déchets par exemple, dit « landfill gas » en anglais), ou bien issu d'une contamination naturelle (gaz dit « marsh gas » ou bien « stray gas » en anglais), ou encore un gaz produit naturellement (tel que du méthane).

Ainsi, l'information contenue dans la composition géochimique des gaz dissous dans un réservoir d'eau peut permettre de renseigner sur l'origine d'une ressource gazeuse et la teneur en cette ressource. L'origine et la teneur d'une espèce gazeuse dissoute dans l'eau d'un réservoir sont des informations essentielles pour (1) envisager l'exploitation de l'espèce en question ou (2) comprendre la contamination du réservoir par une activité industrielle (pollution d'un aquifère par exemple) ou par un processus naturel (sismicité, thermalisme, etc...). Par ailleurs, la charge en gaz de réservoirs d'eau peut être une information essentielle pour la production de réservoir d'énergie thermique (géothermie).

Une application particulière de la présente invention concerne le stockage géologique de $CO_2$ dans une formation souterraine. La directive européenne 2009/31/CE exige un stockage permanent et sûr pour l'environnement, prévenant et maîtrisant les remontées de $CO_2$ et de substances annexes vers la surface, tout en limitant les perturbations du milieu souterrain. Ainsi, un taux de fuite de $CO_2$ de 0,01% par an au droit d'un site de séquestration géologique de $CO_2$ est au maximum toléré selon cette directive. Afin d'être conforme aux réglementations en vigueur, et aussi pour contribuer à l'acceptation sociétale de cette technologie, il apparait nécessaire de mettre au point des techniques de surveillance des sites de stockage géologique de $CO_2$, permettant de détecter et quantifier d'éventuelles fuites.

## Etat de la technique

Les documents suivants seront cités dans la suite de la description :

Duan, Z., Moller, N., Greenberg, J., Weare, JH., 1992. The prediction of methane solubility in natural waters to high ionic strength from 0 to 250°C and from à to 1600 bar. Geochimica et Cosmochimica Acta, 56, 1451-1460.

Duan, Z., Mao, S., 2006. A thermodynamic model for calculating methane solubility, density, and gas phase composition of methane-bearing aqueous fluids from 273 to 253 K abd from 1 to 2000 bar. Geochimica et Cosmochimica Acta, 70, 3369-3386.

Gevantman, LH., 2003, Solubility of selected gases in water. CRC Handbook of Chemistry and Physics, 8, Pages 86-87.

Gonzalez-Penagos, F., Rouchon, V., Guichet, Moretti, I., Accepted. The distribution of thermogenic, bacterial and inorganic fluid sources in the petroleum systems of the Llanos Basin (Colombia) - Insights from the noble gases and carbon stable isotopes. MARINE AND PETROLEUM GEOLOGY, 71, 391-408.

Johnson RL., Pankow, JF., Cherry, JA., 1987. Design of a Ground-Water Sampler for Collecting Volatile Organics and Dissolved Gases in Small-Diameter Wells.Ground Water, 25-4, 448-454.

Kampbell, DH., Vandegrift, SA., 1998. Analysis of Dissolved Methane, Ethane, and Ethylene in Ground Water by a Standard Gas Chromatographic Technique. journal of Chromatographic Science, 36, 253-256.

Kontogeorgis, GM., Voutsas, EC., Yakoumis, JV., and Tassios, DP., , 1996. An equation of state for associating fluids. Industrial Engineering Chemistry Research, 35, 4310-4318.

Magnier, C., Rouchon, V., Bandeira, C., Goncalves, R., Miller, D., Dino, R., 2011. Surface and Subsurface Geochemical Monitoring of an EOR-CO2 Field: Buracica, Brazil. OIL & GAS SCIENCE AND TECHNOLOGY-REVUE D IFP ENERGIES NOUVELLES, 67 (2), 355-372.Soreide, I. and Whitson, C., 1992. Peng-Robinson predictions for hydrocarbons, CO2, N2 and H2S with pure water and NaCI brine. Fluid Phase Equilibria, 77, 217-240.

Sundaram, B., Feitz, A., Caritat, P. de, Plazinska, A., Brodie, R., Coram, J. and Ransley, T., 2009. Groundwater Sampling and Analysis - A Field Guide. Geoscience Australia, Record 2009/27 95 pp.

Whitson, CH., 1988. Fluid Sampling and analysis of laboratory data. Norsk Hydro PVT Analyses Manual, Chapter 3 Fluid Sampling and Laboratory data,

Les méthodes connues à ce jour pour quantifier la concentration en une espèce de gaz présente sous forme dissoute dans un réservoir d'eau d'une formation souterraine nécessitent le plus souvent un prélèvement d'un échantillon liquide directement au sein du réservoir d'eau. On peut citer par exemple la technique qui consiste à réaliser un prélèvement de fond, suivi d'une étude PVT (voir par exemple Johnson et al., 1987, Whitson, 1988) ou bien une analyse des gaz dissous sur un prélèvement d'eau en surface (voir par exemple Sundaram et al., 2009, Kampbell et Vandegrift, 1998). Ces techniques présentent le fort inconvénient de nécessiter un prélèvement de fond ou de surface permettant la préservation des conditions de fond lorsque l'échantillon est remonté à la surface, ce qui peut être difficile à mettre en œuvre techniquement (il faut notamment s'assurer de la non contamination du prélèvement par les fluides présents dans le puits, par l'air en surface, ainsi que se prémunir du fractionnement compositionnel des gaz dissous entre les phases liquide et vapeur lors de la décompression du fluide) et qui peut se révéler onéreux, particulièrement lorsque le prélèvement doit être répété dans le temps (cas de la surveillance d'un site stockage géologique d'un gaz par exemple où un dispositif de prélèvement doit être descendu de façon répétée dans le puits foré).

On connaît également une technique (voir par exemple Whitson, 1998) qui consiste à déterminer la concentration en une espèce de gaz présente sous forme dissoute dans un réservoir d'eau en combinant une analyse de gaz prélevés en surface de la formation d'intérêt et des tests de puits, notamment des mesures de débits d'eau et de gaz à la surface du puits. Si cette technique présente le fort avantage de ne pas nécessiter de prélèvements directement au sein du réservoir d'eau d'intérêt, elle requiert néanmoins l'existence d'au moins un puits, et de réaliser des tests de puits, notamment des mesures de débits d'eau et de gaz à la surface du puits. La mise en oeuvre de cette technique nécessitant des essais de puits peut donc être complexe et onéreuse, particulièrement lorsque les essais de puits doivent être répétés dans le temps (cas de la surveillance d'un site stockage géologique d'un gaz par exemple où des essais de puits doivent être réalisés de façon répétée dans le temps).

On connait également le document :

The noble gas geochemistry of natural CO2 gas reservoirs from the Colorado Plateau and Rocky Mountain provinces, USA, Gilfillan, Stuart; Ballentine, Chris J.; Holland, Greg; Blagburn, Dave; Lollar, Barbara Sherwood; Stevens, Scott; Schoell, Martin; Cassidy, Martin. In: Geochimica et Cosmochimica Acta, Vol. 72, No. 4, 15.02.2008, p. 1174-1198, qui décrit une méthode pour identifier la source de CO2 dans des réservoirs à partir de gaz rares et le développement de modèles physiques pour prendre en compte la migration et l'interaction du CO2 avec l'eau d'un réservoir.

La présente invention vise à déterminer la concentration en une espèce de gaz d'intérêt dans un réservoir d'eau de manière simple, non invasive et peu onéreuse. En particulier le procédé selon l'invention ne nécessite pas nécessairement un forage (cas d'une résurgence naturelle par exemple), et en tout état de cause, ne nécessite pas de prélèvement de fond ni d'essai de puits. En effet, la présente invention repose sur une analyse de gaz issus du dégazage (naturel ou artificiel) d'un réservoir d'eau souterraine et prélevés en surface afin de pouvoir déterminer quantitativement la composition en gaz dissous au sein du réservoir.

Le procédé selon l'invention permet notamment de surveiller plus simplement la contamination naturelle ou industrielle par différentes espèces gazeuses de ces eaux situées à proximité de sites d'exploration ou d'exploitation de ressources telles que des activités de stockage de gaz, de production de gaz, des activités de géothermie, ou autres activités.

## Le procédé selon l'invention

L'invention concerne un procédé pour l'exploitation et/ou la surveillance d'une formation souterraine comportant au moins un réservoir comprenant de l'eau, au moins une espèce de gaz et au moins un isotope atmosphérique d'au moins un gaz rare étant présentes sous forme dissoute dans l'eau dudit réservoir. Le procédé selon l'invention est défini par les revendications 1 à 6.

D'autres caractéristiques et avantages du procédé selon l'invention apparaîtront à la lecture de la description ci-après.

## Description détaillée du procédé

De façon générale, l'invention concerne un procédé pour l'exploitation et/ou la surveillance d'une formation souterraine comportant au moins un réservoir comprenant de l'eau, au moins une espèce de gaz d'intérêt et au moins un gaz rare atmosphérique étant présents sous une forme dissoute dans l'eau du réservoir.

La présente invention vise en particulier à déterminer la concentration en l'espèce de gaz d'intérêt dans l'eau du réservoir. Le gaz d'intérêt que l'on souhaite soit exploiter soit surveiller peut être de type hydrocarbure gazeux (tel que du méthane), du CO2, de l'hydrogène, de l'acide sulfurique, etc.

Le procédé selon l'invention repose sur l'hypothèse que le réservoir d'eau souterraine a été alimenté naturellement par une eau en équilibre de solubilité avec l'air, aux conditions de pression et de températures ambiantes. Cela signifie en particulier que le réservoir d'eau étudié est initialement (c'est-à-dire avant tout dégazage) en équilibre de solubilité avec l'air, ou dit autrement que la teneur initiale en gaz dissous dans le réservoir (c'est-à-dire la teneur en gaz dissous dans le réservoir d'eau avant dégazage, naturel ou artificiel) correspond à l'équilibre de solubilité dans l'eau à pression et température ambiante des composés atmosphériques. Il est à noter que cette hypothèse satisfait le cas le plus général des aquifères.

Par ailleurs, le procédé selon l'invention repose sur l'hypothèse que la phase vapeur issue du dégazage de l'eau présente dans le réservoir d'eau souterraine a été produite à l'équilibre de solubilité avec le réservoir d'eau souterraine.

En outre, le procédé selon l'invention repose sur une référence compositionnelle correspondant à la teneur en au moins un isotope atmosphérique d'un gaz rare, assimilée à l'équilibre de l'eau avec l'air aux conditions de pression et de températures ambiantes. Par isotope atmosphérique d'un gaz rare, on entend un isotope d'un gaz rare dont la présence dans le réservoir d'eau souterraine ne peut qu'être issu d'un équilibre avec l'atmosphère. On citera par exemple les isotopes atmosphériques suivants : l'isotope $^{20}$Ne pour le Néon, l'isotope $^{36}$Ar pour l'Argon et l'isotope $^{84}$Kr pour le Krypton.

Ces hypothèses permettent une détermination de la concentration en l'espèce de gaz d'intérêt dans l'eau du réservoir, de manière représentative des phénomènes physiques (en gardant la réalité physique sous-jacente...), tout en restant simple d'utilisation.

Sur la base de ces hypothèses, on peut alors considérer que tout composé en excès par rapport à une référence compositionnelle qui serait d'origine atmosphérique correspond à l'ajout d'un constituant dans le réservoir d'eau. En effet, le réservoir d'eau étant en équilibre de solubilité avec l'air, les isotopes atmosphériques des gaz rares présents dans l'eau ne peuvent être qu'issus de l'équilibre de l'eau avec l'air. Par conséquent, la teneur initiale en isotope atmosphérique correspond à l'équilibre de solubilité des composés atmosphériques dans l'eau à pression et température ambiante. Ainsi, on est en mesure d'appliquer valablement la loi de Henry, bien connue du spécialiste, afin de déterminer la concentration en gaz atmosphériques dans l'eau. En effet, la loi de Henry définit l'équilibre de solubilité selon la constante de Henry $K_i$ pour un composé $i$, en fonction de la concentration de ce composé dans les phases vapeur ($C^i_v$) et liquide ($C^i_l$), soit :

$$C^i_v = C^i_l.\, K_i \qquad\qquad (1)$$

L'air étant un réservoir infini et de composition fixe, le paramètre $C^i_v$ est connu pour tous les composés atmosphériques. De plus, les coefficients de Henry pour les composés de l'air en fonction de la température d'équilibre sont bien connus des spécialistes (on pourra par exemple se référer au document Gevantman, 2003). Ainsi, il est possible de déterminer la concentration en gaz atmosphériques dans l'eau $C^i_l$ à partir de l'application de la loi de Henry.

De plus, les gaz rares étant intrinsèquement inertes chimiquement et biologiquement, leur concentration dans l'eau ne peut évoluer qu'au grès de processus physiques tels que la diffusion, le mélange et les équilibres de phase. Ainsi, la concentration dans un réservoir d'eau sous-terraine en isotopes $^{20}$Ne, $^{36}$Ar et $^{84}$Kr ne peut évoluer que si le réservoir d'eau sous-terraine devient assimilable à un système ouvert en libre échange avec une réservoir de fluide non équilibré avec l'air aux conditions ambiantes (ce qui se produit par exemple lorsque l'eau est mise au contact d'une eau non équilibrée avec l'air, lorsque l'eau a interagi avec un fluide non équilibré avec l'air, ou lorsque l'eau a subi un changement de phase, notamment la formation d'une phase vapeur). Si un réservoir d'eau n'a jamais été soumis à une des trois conditions listées ci-dessus, sa concentration en gaz atmosphériques est prédictible via la loi de Henry.

Il est bien évident pour le spécialiste que les hypothèses énoncées ci-dessus ne sont pas limitatives, et que l'application du procédé selon l'invention à un réservoir d'eau qui ne satisferait pas pleinement au moins une des hypothèses énoncées ci-dessus est possible. Il est toutefois bien évident que l'application du procédé selon l'invention à un réservoir d'eau qui satisferait au mieux les hypothèses énoncées ci-dessus conduirait à une estimation plus fiable de la concentration en l'espèce de gaz d'intérêt dans l'eau du réservoir.

Le procédé selon l'invention comporte au moins les étapes suivantes :

    **1. Prélèvement d'un échantillon gazeux en surface**
    **2. Analyse de l'échantillon gazeux prélevé**
    **3. Quantification du gaz dissous**

Les étapes principales de la présente invention sont détaillées ci-après.

## 1. Prélèvement d'un échantillon gazeux en surface

Au cours de cette étape, il s'agit de réaliser au moins un prélèvement gazeux au niveau d'au moins une zone de collecte de l'eau du réservoir souterrain, cette zone étant située à la surface de la formation souterraine. Cette collecte de l'eau du réservoir souterrain peut prendre la forme d'une collecte passive lorsque cette eau remonte naturellement du réservoir à la surface par l'effet du gradient de pression, ou bien peut nécessiter un puits, éventuellement équipé d'un système de pompage lorsque la pression naturelle dans le réservoir d'eau est insuffisante pour faire remonter l'eau du réservoir à la surface de manière spontanée.

Le prélèvement gazeux peut être réalisé de différentes manières, selon le type de réservoir d'eau et son moyen de collecte. La contrainte principale à respecter est de préserver le prélèvement de toute contamination à l'air ambiant. Pour ce faire, le contenant de l'échantillon doit être caractérisé par une bonne étanchéité à l'eau et au gaz, et permettre une conservation fiable entre l'instant de l'échantillonnage et l'analyse au laboratoire. Par la suite, on parlera de cylindre d'échantillonnage pour désigner le contenant de l'échantillon.

Selon l'invention, on utilise un cylindre d'échantillonnage constitué d'un corps tubulaire en métal ou en verre, isolé de l'extérieur par une vanne à chaque extrémité.

Selon une application du procédé selon l'invention à un aquifère équipé d'un puits et d'une pompe, ou à un aquifère artésien équipé d'un puits, ou à une résurgence naturelle captée par un tuyau ou un puits, le cylindre d'échantillonnage destiné au prélèvement de l'échantillon gazeux est positionné en tête de puits. Ainsi, le prélèvement est réalisé à la surface, et non pas au fond du puits. Avantageusement, le prélèvement est effectué après balayage du cylindre d'échantillonnage par le flux d'eau engendré par la pompe (dans le cas d'un puits équipé d'une pompe) ou le débit naturel (dans le cas d'un puits artésien).

Selon une application du procédé selon l'invention à une résurgence d'eau naturelle non équipée d'un puits, le cylindre d'échantillonnage comporte un moyen pour isoler de l'air ambiant le flux de gaz provenant de l'eau. Selon une mise en œuvre de l'invention, le cylindre d'échantillonnage comporte un corps tubulaire raccordé à un entonnoir à l'une de ces extrémités, entonnoir qui peut être en verre, métal ou plastique. Pour ce mode de mise en œuvre, on réalise un captage de la résurgence en présentant la partie du cylindre d'échantillonnage formée de l'entonnoir au niveau de la résurgence. De cette manière, le gaz exsolvé de l'eau balaye le sommet de l'entonnoir et le cylindre d'échantillonnage et peut être ainsi prélevé en évitant toute contamination avec l'air ambiant.

## 2. Analyse de l'échantillon gazeux prélevé

Au cours de cette étape, il s'agit d'analyser les gaz prélevés à l'étape précédente. Selon l'invention, on mesure au moins la concentration de l'espèce de gaz considérée (c'est-à-dire par exemple un hydrocarbure gazeux (tel que du méthane), du $CO_2$, de l'hydrogène, de l'acide sulfurique) dans l'échantillon gazeux prélevé à l'étape précédente ainsi que la concentration d'au moins un isotope atmosphérique d'au moins un gaz rare dans ledit échantillon gazeux prélevé à l'étape précédente.

Selon une mise en œuvre de l'invention, on quantifie ladite espèce d'intérêt dans l'échantillon gazeux prélevé par chromatographie en phase gazeuse, par spectrométrie de masse, par analyse infra rouge, électrochimique ou encore par interférométrie Raman. Le spécialiste a parfaite connaissance de la manière de mettre en œuvre de telles techniques de manière à mesurer la concentration en une espèce de gaz d'intérêt. On pourra se référer aux documents (Gonzalez Penagos et al., 2016) et (Magnier et al., 2012) pour de plus amples détails sur des méthodes d'analyse adaptées à la mise en œuvre de l'invention.

Selon une réalisation de l'invention, on détermine la concentration d'un isotope atmosphérique d'un gaz rare dans l'échantillon gazeux prélevé à l'étape précédente par spectrométrie de masse, et ce notamment afin de définir la fraction isotopique proprement atmosphérique de l'espèce considérée Selon un mode de mise en œuvre de l'invention, l'isotope atmosphérique considéré est l'isotope $^{36}Ar$ de l'Argon, ou bien l'isotope $^{20}Ne$ du Néon, ou bien l'isotope $^{84}Kr$ du Krypton. Selon une mise en œuvre de l'invention, on détermine la concentration en chacun des isotopes atmosphériques suivants : isotope $^{36}Ar$ de l'Argon, isotope $^{20}Ne$ du Néon et isotope $^{84}Kr$ du Krypton

Selon un mode de mise en œuvre de l'invention dans lequel un des gaz rares considéré est l'Argon, on détermine la concentration en isotope atmosphérique de l'Argon par une analyse par chromatographie en phase gazeuse couplée à un spectromètre de masse.

Avantageusement, en sus de l'Argon, on mesure la concentration d'au moins un isotope atmosphérique d'un autre gaz rare, tel que le Néon, l'Argon, le Krypton, ou le Xénon.

### 3. Quantification du gaz dissous

Au cours de cette étape, on détermine la concentration de l'espèce de gaz d'intérêt présente sous forme dissoute dans l'eau du réservoir considéré au moyen d'un modèle fonction de la concentration en au moins l'espèce de gaz considérée et de la concentration en au moins un isotope atmosphérique d'un gaz rare présents dans l'échantillon gazeux prélevé à l'étape 1 précédemment décrite, les concentrations précitées ayant été mesurées au cours de l'étape 2 précédemment décrite.

Selon un premier mode de mise en œuvre de l'invention, on utilise un modèle basé sur une méthode analytique pour déterminer la concentration de l'espèce de gaz d'intérêt présente sous forme dissoute dans l'eau du réservoir considéré, à partir des concentrations en au moins l'espèce de gaz considérée et en au moins un isotope atmosphérique présents dans l'échantillon gazeux prélevé.

Alternativement, on utilise un modèle basé sur une méthode numérique pour déterminer la concentration de l'espèce de gaz d'intérêt présente sous forme dissoute dans l'eau du réservoir considéré, à partir des concentrations en au moins l'espèce de gaz considérée et en au moins un isotope atmosphérique présents dans l'échantillon gazeux prélevé. Ces deux modes de mise en œuvre sont déclinés ci-après.

Par la suite, on utilise les notations suivantes :

- indice i : pour désigner un constituant relatif à un isotope atmosphérique ;
- indice j pour désigner un constituant relatif à l'espèce de gaz d'intérêt ;
- exposant *l* pour désigner un constituant sous sa forme liquide ;
- exposant v pour désigner un constituant sous sa forme vapeur.

### 3.1. Méthode analytique

La mise en œuvre du procédé selon l'invention au moyen d'un modèle basé sur une méthode analytique repose sur l'hypothèse selon laquelle le réservoir d'eau est en équilibre de solubilité avec l'air, ou encore que la phase vapeur de tout composé est générée à l'équilibre, artificiellement ou naturellement, à partir d'une phase liquide.

On définit une approche analytique en considérant, en sus de l'hypothèse, que :

- le système diphasique (liquide et vapeur) satisfait aux principes généraux de conservation de la masse et des équilibres de phase ;
- la quantité totale en espèce i est initialement (c'est-à-dire avant dégazage) sous forme dissoute, et le volume de liquide est constant (seul le volume de gaz change selon le degré de sursaturation du système).

Sur la base de ces hypothèses, on peut définir la concentration $C^j_T$ en gaz d'intérêt dissoute dans l'eau selon une formule du type :

$$C^j_T = (1/K_{Hj} + C^i_{air} / (C^i_v . K_{Hi}{}^{amb}) - 1/K_{Hi}{}^z) . C^j_v \qquad (1)$$

où :

- $K_{Hj}$ est la constante de Henry relative à l'espèce d'intérêt j, connue ;
- $C^i_{air}$ est la concentration de l'isotope atmosphérique i dans l'air mesurée ;
- $C^i_v$ est la concentration de la phase vapeur de l'isotope atmosphérique i mesurée ;
- $K_{Hi}{}^{amb}$ est la constante de Henry aux conditions ambiantes de surface (par défaut, 1 bar et 20 °C), connue ;
- $K_{Hi}{}^Z$ le coefficient de Henry du constituant *i* à la profondeur z, connue ;
- $C^j_v$ est la concentration de l'espèce de gaz d'intérêt sous forme vapeur mesurée.

Selon une mise en œuvre de l'invention dans laquelle le réservoir d'eau est situé à des profondeurs relativement faibles, on peut supposer que $K_{Hi}{}^Z$ est proche de $K_{Hi}{}^{amb,}$ et on simplifie l'équation (1) de la façon suivante :

$$C^j_T = (1/K_{Hj} + (C^i_{air}/C^i_v - 1)/K_{Hi}{}^{amb}) . C^j_v \qquad (2)$$

Ainsi, la méthode analytique et ses variantes de réalisation telles que décrites ci-dessus permettent de déterminer la concentration du système initial (c'est-à-dire avant dégazage, artificiel ou naturel) en espèce j en ne se basant que sur la teneur en composé *i* et *j* dans la phase vapeur mesurée en surface, connaissant à priori les coefficients de Henry pour ces deux constituants, et la concentration dans l'air du composé *i*.

## 3.2. Méthode numérique

La troisième étape du procédé selon l'invention peut être mise en œuvre au moyen d'un modèle basé sur une méthode numérique reposant sur l'hypothèse selon laquelle le réservoir d'eau est en équilibre de solubilité avec l'air, ou encore que la phase vapeur de tout composé est générée à l'équilibre, artificiellement ou naturellement, à partir d'une phase liquide.

La méthode numérique selon une mise en œuvre de l'invention permet de déterminer la concentration de l'espèce de gaz d'intérêt présente sous forme dissoute dans l'eau du réservoir considéré, à partir de la concentration en au moins l'espèce de gaz considérée et de la concentration en au moins un isotope atmosphérique présents dans l'échantillon gazeux prélevé.

De manière générale, la méthode numérique pouvant être mise en œuvre pour la résolution de l'étape 3 du procédé selon l'invention repose sur une méthode inverse itérative, comprenant la résolution d'un problème direct à chaque itération et l'optimisation d'une fonction objectif (qui peut correspondre à la minimisation ou la recherche du zéro de la fonction objectif) mesurant un écart entre des valeurs mesurées et des valeurs calculées par la résolution du problème direct.

### • Résolution du problème direct

Selon une mise en œuvre de l'étape 3 de la présente invention, le problème direct de la méthode numérique est basé sur des équations d'état. Selon une variante de mise en œuvre de l'étape 3 de la présente invention, on utilise une méthode numérique basée sur les équations d'état telles que définies dans les documents (Soreide and Whitson, 1992) et (Kontogeorgis et al., 1996). Ces équations permettent de décrire la composition, la densité et l'état des phases de systèmes composés au moins d'eau ($H_2O$), du composé i et du composé j.

Selon une variante de réalisation de l'invention, on utilise une méthode numérique basée sur les équations d'état telles que décrites dans (Soreide and Whitson, 1992), mais étendues aux gaz rares. En particulier, on met en œuvre l'étape 3 de la présente invention sur la base d'une formule du type :

$$P = \frac{RT}{v-b} - \frac{a\,T}{v^2 + 2bv - b^2} \qquad (3)$$

où :

- P est la pression du système ;
- T est la température du système ;
- v est le volume molaire ;
- b est le covolume du système, qui dépend du covolume individuel de chaque composé ;
- a est une constante de l'équation d'état.

Selon une mise en œuvre de l'invention, la constante a telle que définie ci-dessus décrit les interactions entre les composés, est proportionnelle à la fraction molaire de chaque composé, à leurs propriétés thermodynamiques, et à des paramètres d'interactions binaires. Selon une mise en œuvre de l'invention, on définit des constantes $a^{VAP}$ et $a^{AQ}$ pour respectivement les phases vapeur et liquide du composé considéré selon des formules du type :

$$a^{VAP} = \sum_i \sum_j y_i y_j \sqrt{a_i a_j} \left(1 - k_{ij}^{VAP}\right) = \sum_i \sum_j y_i y_j a_{ij}^{VAP}$$

$$a^{AQ} = \sum_i \sum_j x_i x_j \sqrt{a_i a_j} \left(1 - k_{ij}^{AQ}\right) = \sum_i \sum_j x_i x_j a_{ij}^{AQ}. \qquad (4)$$

avec

$$a_i = 0,45724 \frac{R^2 T_{C,i}^2}{P_{C,i}} \alpha \ T_{r,i}$$

- $T_{C,i}$ est la température critique pour le composé i ;
- $Tr_i$ est la température critique pour le composé i ;
- $\alpha$ est un paramètre de l'équation d'état ;
- $k_{ij}$ sont les paramètres d'interactions binaires, pour la phase vapeur (exposant VAP) et pour la phase liquide (exposant AQ) ;
- $x_i$, $x_j$ et $y_i$, $y_j$ sont les fractions molaires des composés i et j dans les phases vapeur et liquide.

**• Définition de la fonction objectif**

Selon une mise en œuvre de l'invention, on cherche à déterminer la concentration de l'espèce de gaz d'intérêt $C^j_T$ en fonction du rapport entre la concentration $C^i_v$ en isotope atmosphérique de gaz rare *i* mesurée dans l'échantillon prélevé à l'étape 1 et la concentration $C^j_v$ en l'espèce d'intérêt j mesurée à l'étape 2 dans l'échantillon prélevé à l'étape 1. Selon cette mise en œuvre, on peut définir une fonction objectif de la forme :

$$f(C^j_T) = [C^i_v / C^j_v]_m - g(P, T, s, C^j_T) \qquad (5)$$

*où* T est la Température, P la pression, *s* la salinité et $g(P, T, s, C^j_T)$ est une fonction correspondant à un modèle d'équation d'état dont le résultat, pour une valeur donnée de concentration $C^j_T$ en gaz d'intérêt dissoute dans l'eau, correspond à la valeur calculée $[C^i_v / C^j_v]_c$. A chaque itération, cette valeur $[C^i_v / C^j_v]_c$ est comparée à chaque itération à la valeur mesurée $[C^i_v / C^j_v]_m$ qui correspond au rapport entre la concentration $C^i_v$ en isotope atmosphérique de gaz rare *i* mesurée dans l'échantillon prélevé à l'étape 1 et la concentration $C^j_v$ en l'espèce d'intérêt j mesurée à l'étape 2 dans l'échantillon prélevé à l'étape 1.

**• Optimisation de la fonction objectif**

Selon une mise en œuvre de l'invention, l'optimisation de la fonction objectif est résolue par la méthode de dichotomie. La méthode de dichotomie permet, grâce à un algorithme mathématique itératif d'identifier la valeur x telle que *f(x)=0*, où *f une* fonction réelle continue sur un intervalle [*Vmin, Vmax*] et *Vmin* et *Vmax* sont deux nombres réels. Pour ce faire, à une itération donnée, la méthode par dichotomie scinde l'intervalle de l'itération courante et conserve, en vue de l'itération suivante, celui des deux sous-intervalles dans lequel existe un zéro de la fonction. Avantageusement, les réels *Vmin* et *Vmax* sont initialisés de sorte que *f(Vmin)* et *f(Vmax)* soient de signe opposé.

Selon un mode de mise en œuvre de l'invention basé sur une fonction objectif *f* définie selon la forme de l'équation (4) décrite ci-dessus, l'identification de *f(C^j_T) = 0* correspond à l'identification du jeu de paramètres (P, T, s, $d_T$) satisfaisant la valeur mesurée dans l'échantillon. Les variables P, T et s étant fixes et données par l'utilisateur, seule la variable $C^j_T$ est à retrouver par la méthode de dichotomie. Pour ce faire, on définit un domaine de variation [*Vmin, Vmax*] relatif à la variable $C^j_T$, et on recherche, par la méthode de dichotomie, la valeur de la variable $C^j_T$, à l'intérieur de cet intervalle, permettant de minimiser la fonction objectif. Avantageusement, ce processus itératif est arrêté lorsqu'un critère d'arrêt prédéfini est atteint. Ce critère peut être correspondre à une valeur maximale acceptable de la fonction objectif (par exemple le processus itératif est arrêté lorsque l'estimation de la fonction objectif est en deçà d'un seuil prédéfini par le spécialiste), ou encore à un nombre maximum d'itérations du processus itératif. Selon une alternative de mise en œuvre de l'invention, on définit un critère d'arrêt basé sur une valeur maximale acceptable de l'écart relatif de la fonction *f* entre les valeurs [*Vmin, Vmax*] de l'itération courante. On peut ainsi définir un seuil noté $\varepsilon$ et arrêter le processus itératif lorsque, à une itération donnée, *(f(Vmin)-f(Vmax))/f(Vmin)< $\varepsilon$*. Par exemple, on peut définir $\varepsilon$ = 0,05 pour une tolérance à la convergence de l'algorithme de 5%. Ainsi, une fois le critère de convergence atteint, la solution obtenue par la méthode numérique reproduit bien la composition totale en espèce i du système initial, ainsi que les compositions, densités et volumes molaires des phases vapeur et liquide de l'espèce j à pression, température et salinité données.

Ainsi, à l'issue de cette étape, qu'elle ait été résolue par une méthode analytique, par une méthode numérique, on obtient la concentration de l'espèce de gaz d'intérêt présente sous forme dissoute dans l'eau du réservoir considéré, à partir de la concentration en au moins l'espèce de gaz considérée et de la concentration en au moins un isotope atmosphérique présents dans l'échantillon gazeux prélevé à l'étape 1, les concentrations précitées ayant été mesurées au cours de l'étape 2.

La concentration en un gaz d'intérêt au sein d'un réservoir d'eau est une information précieuse pour décider d'une exploitation de l'espèce en question, ou encore dans le cas de la surveillance d'un réservoir, pour comprendre la contamination du réservoir par une activité industrielle (pollution d'un aquifère par exemple) ou par un processus naturel (sismicité, thermalisme, etc...). Par ailleurs, la charge en gaz de réservoirs d'eau peut être une information essentielle pour la production de réservoir d'énergie thermique (géothermie).

## Détermination de l'état de phase

Il s'agit d'une étape optionnelle qui consiste à déterminer l'état de phase de l'espèce de gaz considéré au sein du réservoir d'eau considéré, à partir de la concentration en l'espèce de gaz j et de sa saturation dans l'eau. Autrement dit, il s'agit de déterminer si l'espèce d'intérêt j est présente sous forme monophasique ou diphasique dans le réservoir d'eau considéré.

Selon un mode de mise en œuvre de l'invention dans lequel le gaz d'intérêt est le méthane, on peut utiliser le modèle décrit dans les documents (Duan et al., 1992 ; Duan et al., 2006) afin de définir la saturation en gaz d'intérêt j dans l'eau.

Selon une mise en œuvre de l'invention selon laquelle on considère que la loi des gaz parfaits est applicable et que la pression $P$ évolue avec la profondeur z *selon une loi du type : P=P₀+ρ.g.z,* avec $P_0$ la pression atmosphérique, p la masse volumique de l'eau *(ρ= 1,0×10³Kg/m³)*, g l'attraction gravitationnelle *(g = 9.81 m²/s)* et z la hauteur d'eau (en mètres) depuis la surface jusqu'au réservoir d'eau sous-terraine considéré, alors on peut déterminer la saturation $S_j$ en gaz d'intérêt j dans l'eau du réservoir d'eau souterraine selon une formule du type :

$$S_j = [(1/K_{Hj} + (C^i_{air}/ C^i_v - 1) / K^{amb}_{Hi}).C^j_v - C^j_{sat}].R(T_0 + z.G_T)/(\rho.g.z + P_0) \quad (6)$$

où :

- $K_{Hj}$ est la constante de Henry relative à l'espèce d'intérêt j connue;
- $C^i_{air}$ *est* la concentration de l'isotope atmosphérique i dans l'air connue ou mesurée;
- $C^i_v$ est la concentration de la phase vapeur de l'isotope atmosphérique i mesuré dans l'échantillon prélevé ;
- $K_{Hi}^{amb}$ est la constante de Henry aux conditions ambiantes de surface (par défaut, 1 bar et 20 °C) connue ;
- $K_{Hi}^Z$ le coefficient de Henry du constituant *i* à la profondeur z connue ;
- $C^j_v$ est la concentration de l'espèce de gaz d'intérêt j sous forme vapeur
- $C^j_v$ est la concentration de l'espèce de gaz d'intérêt j à saturation pour un couple (P,T) donné.

A partir de la saturation $S_j$ en gaz d'intérêt j dans l'eau du réservoir d'eau souterraine, on peut en déduire l'état de phase du système en considérant que la sursaturation en l'espèce j correspond à une concentration excédentaire $C^j_{exc}$ de cette espèce, soit $C^j_{exc} = C^j_T - C^j_{sat.}$

Après la détermination de la concentration en l'espèce de gaz d'intérêt dans l'eau du réservoir, on peut mettre en œuvre une étape d'exploitation de la formation sous-terraine en fonction de la concentration déterminée, par exemple, le forage d'un nouveau puits, l'injection d'un fluide par un puits injecteur, ...

Alternativement, dans le cadre de la surveillance d'une formation souterraine, par exemple un site de stockage de $CO_2$, on peut réaliser une étape corrective ou préventive pour éviter toute contamination. Par exemple, cette étape peut consister en la remédiation d'un puits.

## Exemple d'application

Les caractéristiques et avantages du procédé selon l'invention apparaîtront plus clairement à la lecture de l'exemple d'application ci-après.

Pour cet exemple, on considère un réservoir d'eau d'une formation souterraine correspondant à un aquifère riche en méthane situé à 400 mètres de profondeur et équipé d'un puits. Au cours de la remontée des eaux de l'aquifère en surface via le puits foré, celles-ci se dégazent et génèrent une phase vapeur. Un échantillon de gaz est prélevé en tête de puits au moyen d'un cylindre d'échantillonnage en inox isolé par des vannes haute pression. Le prélèvement est réalisé aux conditions ambiantes (10°C, 1 bar). Pour la mise en œuvre du procédé selon l'invention, on utilise l'isotope atmosphérique ³⁶Ar de l'Argon, et le gaz d'intérêt dont on cherche à déterminer la concentration dans l'aquifère est le méthane.

L'analyse de cet échantillon est réalisée par chromatographie en phase gazeuse pour déterminer la composition en méthane du gaz prélevé, ainsi que par spectrométrie de masse pour en déterminer la concentration en ³⁶Ar. Les concentrations obtenues pour le méthane et l'isotope atmosphérique ³⁶Ar de l'Argon sont de $y_{CH4}$=92,8 vol.% et $y^{36}Ar$=3,5

×10⁻⁴ vol.% respectivement.

Selon une première mise en œuvre du procédé selon l'invention, on utilise un modèle basé sur une méthode numérique. Plus précisément, afin de remonter à une information correspondant à l'état compositionnel du système dans l'aquifère, on met en œuvre une variante de la méthode numérique basée sur une extension du modèle de Soreide & Whitson (1992) telle que décrite à la sous-étape 3.2. Pour ce faire, on définit la pression hydrostatique de l'aquifère (donc sa profondeur et sa charge artésienne le cas échéant) et on prend la teneur en isotope ³⁶Ar déterminée précédemment comme référence compositionnelle assimilée à l'équilibre de l'eau avec l'air à 1 bar et à une température de surface moyenne annuelle (10°C), sans excès d'air, et sans oxygène.

La variante de la méthode numérique utilisée permettant de fidèlement calculer le fractionnement de ce constituant de référence entre la phase liquide et la phase vapeur pour tous les équilibres, la concentration du ³⁶Ar atmosphérique dans la phase vapeur (donc la phase que nous analysons dans cette étude) permet de déduire l'abondance de la composante à l'équilibre dans l'aquifère, par rapport à une composante dite excédentaire. La composante excédentaire dont on vise ici la quantification est le méthane.

L'application de la variante de mise en œuvre de la méthode numérique telle que décrite ci-dessus permet d'obtenir un nombre de mole total de méthane présent au sein de l'aquifère de 11.9 mol.m⁻³.

Par comparaison, l'application d'une variante de la méthode analytique telle que définie par l'équation (2) décrite à la sous-étape 3.1 aboutit à un nombre de mole total de méthane présent au sein de l'aquifère de 12.1 mol.m⁻³.

Ainsi, on peut observer que les deux méthodes, numérique et analytique, prédisent des concentrations en gaz dissous dans le réservoir d'eau étudié très similaires.

Ainsi le procédé selon l'invention permet de déterminer la concentration en un gaz d'intérêt présent dans un réservoir d'eau souterraine à partir d'un échantillonnage réalisé à la surface de la formation, et d'une analyse de cet échantillon, comprenant la mesure de la concentration en ce gaz d'intérêt dans l'échantillon et la concentration en au moins un isotope atmosphérique d'au moins un gaz rare dans cet échantillon.

De manière générale, ce procédé est particulièrement avantageux pour la surveillance de nappes d'eau souterraines, en permettant notamment de s'affranchir des échantillonnages complexes, ou bien des tests de production de puits habituellement pratiqués dans ce contexte. En effet, ce procédé permet de surveiller plus simplement la contamination naturelle ou industrielle par différentes espèces gazeuses de ces eaux situées à proximité de sites d'exploration ou d'exploitation de ressources telles que des activités de stockage de gaz, de production de gaz, des activités de géothermie, ou autres activités.

**Revendications**

1. Procédé pour l'exploitation et/ou la surveillance d'une formation souterraine comportant au moins un réservoir comprenant de l'eau, au moins une espèce de gaz et au moins un isotope atmosphérique d'au moins un gaz rare étant présents sous forme dissoute dans l'eau dudit réservoir, **caractérisé en ce qu'**on réalise au moins les étapes suivantes :

   i- on réalise au moins un prélèvement d'un échantillon gazeux au niveau d'une zone de collecte de l'eau dudit réservoir située à la surface de ladite formation, ledit prélèvement étant réalisé de manière à éviter toute contamination à l'air au moyen d'un cylindre d'échantillonnage constitué d'un corps tubulaire en métal ou en verre, isolé de l'extérieur par une vanne à chaque extrémité ;
   ii- on mesure au moins la concentration d'au moins ladite espèce de gaz et la concentration d'au moins ledit isotope atmosphérique dudit gaz rare dans ledit échantillon gazeux ;
   iii- on détermine la concentration de ladite espèce de gaz présente dans ledit réservoir sous forme dissoute au moyen d'un modèle de ladite concentration de ladite espèce de gaz sous forme dissoute, ledit modèle étant fonction de ladite concentration d'au moins ladite espèce de gaz dans ledit échantillon gazeux et de ladite concentration dudit isotope atmosphérique dans ledit échantillon gazeux,

   dans lequel ledit modèle est basé sur une méthode analytique reposant sur une formule du type :

$$C^j_T = (1/K_{Hj} + C^i_{air} / (C^i_v . K_{Hi}^{amb}) - 1/K_{Hi}^z) . C^j_v$$

   où :

   - $C^j_T$ est ladite concentration en ladite espèce de gaz d'intérêt j ;
   - $K_{Hj}$ est la constante de Henry relative à ladite espèce de gaz d'intérêt j ;

- $C^i_{air}$ est la concentration dudit isotope atmosphérique i dans l'air ;
- $C^i_v$ est ladite concentration dudit isotope atmosphérique i mesurée dans ledit échantillon ;
- $K_{Hi}^{amb}$ est la constante de Henry relative audit isotope atmosphérique i pour des conditions ambiantes de surface ;
- $K_{Hi}^z$ est le coefficient de Henry dudit isotope atmosphérique i à la profondeur z ;
- $C^j_v$ est ladite concentration de l'espèce de gaz d'intérêt j mesurée dans ledit échantillon,

ou bien ledit modèle est basé sur une méthode numérique itérative comprenant la résolution d'un problème direct à chacune des itérations de ladite méthode numérique itérative et l'optimisation d'une fonction objectif mesurant un écart entre lesdites concentrations mesurées et des concentrations calculées par ladite résolution dudit problème direct.

2. Procédé selon la revendication 1, dans lequel ledit isotope atmosphérique dudit gaz rare est l'isotope $^{20}$Ne du Néon, l'isotope $^{36}$Ar de l'Argon ou l'isotope $^{84}$Kr du Krypton.

3. Procédé selon l'une des revendications précédentes, dans lequel ledit procédé est appliqué à un aquifère équipé d'un puits et d'une pompe, ou à un aquifère artésien équipé d'un puits, ou à une résurgence naturelle captée par un tuyau ou un puits et dans lequel ledit prélèvement est réalisé au moyen dudit cylindre d'échantillonnage étanche placé en tête dudit puits.

4. Procédé selon l'une des revendications 1 à 2, dans lequel ledit procédé est appliqué à une résurgence naturelle de l'eau dudit réservoir en surface non équipée d'un puits, et dans lequel on réalise ledit prélèvement au moyen dudit cylindre d'échantillonnage étanche, ledit corps tubulaire dudit cylindre d'échantillonnage étant raccordé à un entonnoir, ledit échantillon gazeux étant prélevé au niveau dudit entonnoir.

5. Procédé selon l'une des revendications précédentes, dans lequel ledit problème direct est résolu au moyen d'au moins une équation d'état.

6. Procédé selon l'une des revendications précédentes, dans lequel ladite optimisation de ladite fonction objectif est réalisée par une méthode de dichotomie.

**Patentansprüche**

1. Verfahren zur Erschließung und/oder Überwachung einer unterirdischen Formation, die mindestens ein Reservoir beinhaltet, das Wasser umfasst, wobei mindestens eine Gasart und mindestens ein atmosphärisches Isotop mindestens eines seltenen Gases in gelöster Form in dem Wasser des Reservoirs vorliegen, **dadurch gekennzeichnet, dass** mindestens die folgenden Schritte ausgeführt werden:

   i- Ausführen mindestens einer Entnahme einer gasförmigen Probe auf Höhe eines Sammelbereichs des Wassers des Reservoirs, der an der Oberfläche der Formation gelegen ist, wobei die Entnahme so, dass jede Kontamination an der Luft verhindert wird, mittels eines Probenahmezylinders ausgeführt wird, der aus einem rohrförmigen Körper aus Metall oder Glas besteht und der vom Außenraum durch ein Ventil an jedem Ende isoliert ist;
   ii- Messen mindestens der Konzentration mindestens der Gasart und der Konzentration mindestens des atmosphärischen Isotops des seltenen Gases in der gasförmigen Probe;
   iii- Bestimmen der Konzentration der in dem Reservoir in gelöster Form vorliegenden Gasart mittels eines Modells der Konzentration der Gasart in gelöster Form, wobei das Modell von der Konzentration mindestens der Gasart in der gasförmigen Probe und von der Konzentration des atmosphärischen Isotops in der gasförmigen Probe abhängig ist,
   wobei das Modell auf einer analytischen Methode basiert, die auf einer Formel des folgenden Typs beruht:

$$C^j_T = (1/K_{Hj} + C^i_{air} / (C^i_{v*} K_{Hi}^{amb}) -1/ K_{Hi}^z).C^j_v$$

worin:

   - $C^j_T$ die Konzentration der Gasart von Interesse j ist;

- $K_{Hj}$ die Henry-Konstante für die Gasart von Interesse j ist;
- $C^i_{air}$ die Konzentration des atmosphärischen Isotops i in der Luft ist;
- $C^i_v$ die Konzentration des atmosphärischen Isotops i ist, die in der Probe gemessen wurde;
- $K_{Hi}{}^{amb}$ die Henry-Konstante für das atmosphärische Isotop i für Oberflächenumgebungsbedingungen ist;
- $K_{Hi}{}^Z$ der Henry-Koeffizient des atmosphärischen Isotops i in der Tiefe z ist;
- $C^j_v$ die Konzentration der Gasart von Interesse j ist, die in der Probe gemessen wurde,

oder aber das Modell auf einer iterativen numerischen Methode basiert, welche die Lösung eines direkten Problems bei jeder der Iterationen der iterativen numerischen Methode und die Optimierung einer Zielfunktion, die eine Abweichung zwischen den gemessenen Konzentrationen und den durch die Lösung des direkten Problems berechneten Konzentrationen misst, umfasst.

**2.** Verfahren nach Anspruch 1, bei dem das atmosphärische Isotop des seltenen Gases das Isotop $^{20}$Ne von Neon, das Isotop $^{36}$Ar von Argon oder das Isotop $^{84}$Kr von Krypton ist.

**3.** Verfahren nach einem der vorhergehenden Ansprüche, bei dem das Verfahren auf einen Grundwasserleiter angewandt wird, der mit einem Brunnen und einer Pumpe ausgestattet ist, oder auf einen artesischen Grundwasserleiter, der mit einem Brunnen ausgestattet ist, oder auf einen natürlichen Austritt, der mit einem Rohr oder einem Brunnen gefasst ist und in dem die Entnahme mittels des dichten Probenahmezylinders ausgeführt wird, der am Brunnenkopf angeordnet wird.

**4.** Verfahren nach einem der Ansprüche 1 bis 2, bei dem das Verfahren auf einen natürlichen Austritt des Wassers des Reservoirs an der Oberfläche, der nicht mit einem Brunnen ausgestattet ist, angewandt wird und bei dem die Entnahme mittels des dichten Probenahmezylinders ausgeführt wird, wobei der rohrförmige Körper des Probenahmezylinders an einen Trichter angeschlossen ist, wobei die gasförmige Probe auf Höhe des Trichters entnommen wird.

**5.** Verfahren nach einem der vorhergehenden Ansprüche, bei dem das direkte Problem mittels mindestens einer Zustandsgleichung gelöst wird.

**6.** Verfahren nach einem der vorhergehenden Ansprüche, bei dem die Optimierung der Zielfunktion mit einer Dichotomiemethode ausgeführt wird.

## Claims

**1.** Method for exploiting and/or monitoring an underground formation comprising at least one reservoir containing water, at least one gas species and at least one atmospheric isotope of at least one rare gas being present in dissolved form in the water of said reservoir, **characterized in that** at least the following steps are carried out:

i- at least one gas sample is taken at a water collecting zone of said reservoir, which zone is situated at the surface of said formation, said sample being taken in such a way as to avoid any contamination with the air, using a sampling cylinder made up of a tubular body made of metal or of glass, isolated from the outside by a valve at each end;

ii- at least the concentration of at least said gas species and the concentration of at least said atmospheric isotope of said rare gas in said gas sample are measured;

iii-the concentration of said gas species present in said reservoir in dissolved form is determined by means of a model of said concentration of said gas species in dissolved form, said model being a function of said concentration of at least said gas species in said gas sample and of said concentration of said atmospheric isotope in said gas sample,

wherein said model is based on an analytical method relying on a formula of the type:

$$C^j_T \ = \ (1/K_{Hj} \ + \ C^i_{air}/(C^i_v . K_{Hi}{}^{amb}) - 1/K_{Hi}{}^z) . C^j_v$$

where:

- $C^j_T$ is said concentration of said gas species of interest j;
- $K_{Hj}$ is the Henry constant relating to said gas species of interest j;
- $C^i_{air}$ is the concentration of said atmospheric isotope i in the air;
- $C^i_v$ is said concentration of said atmospheric isotope i measured in said sample;
- $K_{Hi}{}^{amb}$ is the Henry constant relating to said atmospheric isotope i for surface ambient conditions;
- $K_{Hi}{}^Z$ is the Henry coefficient of said atmospheric isotope i at the depth z;
- $C^i_v$ is said concentration of the gas species of interest j measured in said sample,

or else said model is based on an iterative numerical method involving resolving a direct problem on each of the iterations of said iterative numerical method and optimizing an objective function measuring a discrepancy between said measured concentrations and concentrations calculated by said resolution of said direct problem.

2. Method according to Claim 1, wherein said atmospheric isotope of said rare gas is the isotope $^{20}$Ne of neon, the isotope $^{36}$Ar of argon, or the isotope $^{84}$Kr of krypton.

3. Method according to either of the preceding claims, wherein said method is applied to an aquifer equipped with a well and with a pump, or to an artesian aquifer equipped with a well, or to a natural spring captured by a pipe or a well and in which said sample is taken by means of said fluid tight sampling cylinder placed at the head of said well.

4. Method according to either of Claims 1 and 2, wherein said method is applied to a natural spring of water from said reservoir at a surface not equipped with a well and wherein said sample is taken by means of said fluid tight sampling cylinder, said tubular body of said sampling cylinder being connected to a funnel, said gas sample being taken off at said funnel.

5. Method according to one of the preceding claims, wherein said direct problem is resolved by means of at least one state equation.

6. Method according to one of the preceding claims, wherein said optimization of said objective function is performed using a dichotomy method.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **DUAN, Z. ; MOLLER, N. ; GREENBERG, J. ; WEARE, JH.** The prediction of methane solubility in natural waters to high ionic strength from 0 to 250°C and from à to 1600 bar. *Geochimica et Cosmochimica Acta,* 1992, vol. 56, 1451-1460
- **DUAN, Z. ; MAO, S.** A thermodynamic model for calculating methane solubility, density, and gas phase composition of methane-bearing aqueous fluids from 273 to 253 K abd from 1 to 2000 bar. *Geochimica et Cosmochimica Acta,* 2006, vol. 70, 3369-3386
- **GEVANTMAN, LH.** Solubility of selected gases in water. *CRC Handbook of Chemistry and Physics,* 2003, vol. 8, 86-87
- **GONZALEZ-PENAGOS, F. ; ROUCHON, V. ; GUICHET, MORETTI, I.** The distribution of thermogenic, bacterial and inorganic fluid sources in the petroleum systems of the Llanos Basin (Colombia) - Insights from the noble gases and carbon stable isotopes. *MARINE AND PETROLEUM GEOLOGY,* vol. 71, 391-408
- **JOHNSON RL. ; PANKOW, JF. ; CHERRY, JA.** Design of a Ground-Water Sampler for Collecting Volatile Organics and Dissolved Gases in Small-Diameter Wells. *Ground Water,* 1987, vol. 25-4, 448-454
- **KAMPBELL, DH. ; VANDEGRIFT, SA.** Analysis of Dissolved Methane, Ethane, and Ethylene in Ground Water by a Standard Gas Chromatographic Technique. *journal of Chromatographic Science,* 1998, vol. 36, 253-256
- **KONTOGEORGIS, GM. ; VOUTSAS, EC. ; YAKOUMIS, JV. ; TASSIOS, DP.** An equation of state for associating fluids. *Industrial Engineering Chemistry Research,* 1996, vol. 35, 4310-4318
- **MAGNIER, C. ; ROUCHON, V. ; BANDEIRA, C. ; GONCALVES, R. ; MILLER, D. ; DINO, R.** Surface and Subsurface Geochemical Monitoring of an EOR-CO2 Field: Buracica, Brazil. *OIL & GAS SCIENCE AND TECHNOLOGY-REVUE D IFP ENERGIES NOUVELLES,* 2011, vol. 67 (2), 355-372
- **SOREIDE, I. ; WHITSON, C.** Peng-Robinson predictions for hydrocarbons, CO2, N2 and H2S with pure water and NaCl brine. *Fluid Phase Equilibria,* 1992, vol. 77, 217-240
- **SUNDARAM, B. ; FEITZ, A. ; CARITAT, P. DE ; PLAZINSKA, A. ; BRODIE, R. ; CORAM, J. ; RANSLEY, T.** Groundwater Sampling and Analysis - A Field Guide. *Geoscience Australia, Record,* 2009, 95
- Fluid Sampling and analysis of laboratory data. **WHITSON, CH.** Norsk Hydro PVT Analyses Manual. 1988
- **JOHNSON et al.** *Whitson,* 1987
- **GILFILLAN, STUART ; BALLENTINE, CHRIS J. ; HOLLAND, GREG ; BLAGBURN, DAVE ; LOLLAR, BARBARA SHERWOOD ; STEVENS, SCOTT ; SCHOELL, MARTIN ; CASSIDY, MARTIN.** The noble gas geochemistry of natural CO2 gas reservoirs from the Colorado Plateau and Rocky Mountain provinces. *Geochimica et Cosmochimica Acta,* 15 Février 2008, vol. 72 (4), 1174-1198